# EUROPEAN PATENT SPECIFICATION

(11) **EP 3 886 792 B1**
(45) Date of publication and mention of the grant of the patent: **16.09.2026**
(21) Application number: 19789706.9
(22) Date of filing: 23.10.2019
(51) Int. Cl.: A61K 8/31, A61K 8/34, A61K 8/37, A61K 8/58, A61Q 15/00, A61K 8/04

(54) **NON-ALUMINIUM ANTIPERSPIRANT COMPOSITIONS**
ALUMINIUMFREIE SCHWEISSHEMMENDE ZUSAMMENSETZUNGEN
COMPOSITIONS ANTISUDORIFIQUES SANS ALUMINIUM

(30) Priority: 30.11.2018 EP 18209514
(43) Date of publication of application: 06.10.2021
(73) Proprietor: UNILEVER GLOBAL IP LIMITED, Wirral Merseyside CH62 4ZD (GB); Unilever IP Holdings B.V., 6708 WH Wageningen (NL)
(72) Inventor: ZDRAVKOVA, Aneliya, Nikolova, Wirral Merseyside CH63 3JW (GB)
(74) Representative: Unilever Patent Group
(86) International application number: PCT/EP2019/078928
(87) International publication number: WO 2020/108882

(56) References cited:
- WO-A1-94/24993
- GB-A- 2 273 872
- US-A1- 2008 267 895
- US-A1- 2010 104 517
- US-A1- 2016 045 406

## Description

### Field of Invention

The present invention is in the field of cosmetic compositions and their use as antiperspirants, in particular, non-aluminium antiperspirant compositions.

### Background

EP 550,960 A1 (Unilever, 1992) discloses the use as an antiperspirant active of an amphiphilic material which forms, upon contact with perspiration, a water-insoluble liquid crystal phase of greater than one dimensional periodicity. This publication does not disclose ethanolic compositions, nor their stability issues, nor their use in aerosol compositions.

WO 94/024993 (Unilever, 1994) discloses an antiperspirant composition comprising an amphiphilic material which forms, upon contact with perspiration, a water-insoluble liquid crystal phase of greater than one dimensional periodicity, in a cosmetic vehicle comprising a volatile silicone and containing less than 10% by weight of the total composition of a short chain monohydric alcohol. This publication does not disclose compositions with the specific ratios of components as defined herein and does not disclose their importance in the low temperature (0°C) stability thereof.

### Summary of Invention

It is an object of the present invention to provide an antiperspirant aerosol composition that does not require the presence of an aluminium or zirconium salt to deliver an antiperspirancy benefit. It is a further object of the present invention to do this from an aerosol composition that has a high degree of storage stability, particularly at low temperatures.

In a first aspect of the invention, there is provided an antiperspirant aerosol composition comprising ethanol, amphiphilic material, volatile silicone and a hydrocarbon propellant, the amphiphilic material being a mixture consisting of isostearyl alcohol and glycerol monolaurate and at a ratio of from 25: 75 to 45: 55, wherein:
(i) a three-component base composition consisting of the ethanol, amphiphilic material and volatile siloxane consists of 15 to 40% amphiphilic material; 10 to 50% volatile silicone and 12 to 75% ethanol; the sum of these three components being 100%; and
(ii) the ratio of ethanol to volatile silicone is 1: 4 or greater by weight; and
(iii) the propellant comprises from 35 to 95% by weight of the total composition.

In a second aspect of the invention, there is provided a method of manufacture of an antiperspirant aerosol composition comprising the addition of a hydrocarbon propellant to a composition comprising all the other components referred to in the first aspect of the invention.

In a third aspect of the invention, there is provided a cosmetic method of attaining an antiperspirant benefit comprising the topical application of a composition according to the first aspect of the invention.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, "ambient conditions" refers to about 20°C and 1 atmosphere (1013.25 hPa) pressure, unless otherwise indicated.

Herein, all percentages, ratios and amounts are by weight, unless otherwise indicated.

Herein, the word "comprising" is intended to mean "including" but not necessarily "consisting of", i.e., it is non-exhaustive.

Herein, "cosmetic" methods and compositions should be understood to mean non-therapeutic methods and compositions, respectively.

Herein, an "amphiphilic material" is a material defined by having both hydrophilic and hydrophobic portions in its structure.

Herein, "water-insoluble" means having a solubility in water of less than 0.1% by weight (at 37°C).

Herein, the term "hydrocarbon" propellant refers to propellants consisting solely of hydrogen and carbon atoms and having a boiling point of less than 20°C. For the avoidance of doubt, this definition excludes ethers such dimethyl ether.

Herein, references to "the boiling point" refer to the boiling point of the combination of all components covered by the generic descriptor used, when a generic descriptor is employed.

The compositions of the invention are particularly effectively when topically applied to the underarm regions of the human body and/or the feet. The compositions are especially effectively when topically applied to the underarm regions of the human body.

Antiperspirant compositions according to the present invention are preferably free of aluminium or zirconium antiperspirant salts. Indeed, they are more preferably free of any aluminium or zirconium salts.

Herein, "free of" means having less 0.1% and preferably less than 0.01% of the specified component or components.

Antiperspirant aerosol compositions consist of a propellant and a base. The components of the base are typically mixed together first and the propellant is added last in a process sometimes called "gassing". It is important that the base has good storage stability because there can be a significant period between the preparation of the base and the addition of the propellant.

Herein, the "base" of an antiperspirant aerosol composition is all the components of the total composition other than the propellant.

It is important that the fully formulated antiperspirant aerosol composition has good storage stability, so that it can survive prolonged transit to stores and extended periods on shelf prior to purchase and use.

The present invention involves compositions having superior storage stability, including stability at low temperatures, such as 0°C. By achieving stability at both ambient and low temperatures, the present invention enables the delivery of fully formulated antiperspirant aerosol compositions having good storage stability over prolonged periods and an extended range of temperatures.

### Amphiphilic Material

The amphiphilic material is a mixture consisting of isostearyl alcohol and glycerol monolaurate and at a ratio of from 25: 75 to 45: 55. The amphiphilic material forms, upon contact with perspiration, a water-insoluble liquid crystal phase of greater than one dimensional periodicity. The amphiphilic material is capable of forming phases as described immediately above, on contact with perspiration on the skin of the human body, at body temperature (37°C) and preferably at from 25°C to 37°C.

It is essential to have sufficient of the amphiphilic material present in the composition to achieve an acceptable degree of antiperspirancy when the composition is applied to the skin of the human body. The amphiphilic material is at least 15% of the tri-component mixture consisting of ethanol, amphiphilic material and volatile siloxane. The maximum content of amphiphilic material in the tri-component mixture consisting of ethanol, amphiphilic material and volatile siloxane is 40% and preferably 30%, for reasons of formulation stability.

The amphiphilic material serves as the antiperspirant active for the composition, just as in the prior publications EP 550,960 A1 and WO 94/024993 by Unilever.

According to a preferred embodiment of the invention, the amphiphilic material physically swells and forms a liquid crystal structure on contact with perspiration, hence enhancing the pore-blocking effect.

### Volatile Silicone

Herein, a "volatile silicone" is a silicone having a vapour pressure of greater than 1 Pa at 25°C.

The volatile silicone and its level of incorporation serves to reduce potential irritation and/or promote the good sensory properties of the composition.

The volatile silicone is present at from 10% to 50% and more preferably at from 15 to 30% of the tri-component mixture consisting of ethanol, amphiphilic material and volatile siloxane.

It is preferred that the volatile silicone comprises greater than 90% by weight of, or consists of siloxanes having from 2 to 6 silicone atoms, arranged in either a cyclic or linear fashion. Linear siloxanes have the general formula: Me₃SiO(Me₂SiO)ₙSiMe₃, where Me = methyl group (-CH₃).
When n = 0, the siloxane is hexamethyldisiloxane.
When n = 1, the siloxane is octamethyltrisiiloxane.
When n = 2, the siloxane is decamethyltetrasiloxane.
When n = 3, the siloxane is dodecamethylpentasiloxane.

It is particularly preferred that the volatile silicone comprises greater than 90% by weight of, or consists of, siloxanes selected from the group consisting of hexamethyldisiloxane, octamethyltrisiiloxane, decamethyltetrasiloxane, dodecamethylpentasiloxane and decamethylcyclopentasiloxane.

It is especially preferred that the volatile silicone comprises greater than 90% by weight of, or consists of, siloxanes selected from the group consisting of hexamethyldisiloxane, octamethyltrisiiloxane, decamethyltetrasiloxane and decamethylcyclopentasiloxane.

### Ethanol

Ethanol is an essential component of compositions of the invention. The purpose of the ethanol is principally to solubilise the amphiphilic material, in balance with the volatile silicone which is also an essential component of compositions of the invention.

The ratio of ethanol to volatile silicone is 1: 4 or greater, preferably 1: 3 or greater and more preferably 1: 2 or greater. These ratios equate to 20: 80 or greater, preferably 25: 75 or greater and more preferably 33.3 to 66.7 or greater. These ratios enable the generation of the required (phase) stability for the compositions of the invention.

The upper level for ethanol in the compositions of the invention is dictated by the restrictions previously referred to.

The lower level for ethanol in the compositions of the invention is also dictated by the restrictions previously referred to. Concentrating on the three-component 'base' composition consisting of ethanol, amphiphilic material and volatile siloxane, the maximum content of amphiphilic material is 40% and the minimum ratio of ethanol to volatile silicone is 1: 4. Hence, the minimum content of ethanol in this three-component composition/system is 12%.

Hence, the ethanol is present at from 12% to 75% of the tri-component mixture consisting of ethanol, amphiphilic material and volatile siloxane.

The three-component 'base' composition consisting of ethanol, amphiphilic material and volatile siloxane consists 15 to 40% amphiphilic material, 10 to 50% volatile silicone and 12 to 75% ethanol, the sum of these three components being 100%.

### Hydrocarbon Propellant

An essential component of compositions of the invention is a hydrocarbon propellant. Such materials are by their nature extremely hydrophobic. Surprisingly, we have found that the relatively hydrophilic bases used with the present invention are compatible with this hydrophobic propellant, provided that the ratios of components in the base are carefully selected. Without such selection, multiphasic or unstable compositions result.

To function effectively, the hydrocarbon propellant must have a relatively low boiling point, generally less than 20°C, but preferably less than 10°C and more preferably less than 0°C.

The hydrocarbon propellant preferably comprises from 35 to 95% of the total composition, more preferably from 40 to 90% and most preferably from 50 to 85% of the total composition.

Preferred hydrocarbon propellants include n-butane, isobutane and propane, and blends thereof. A particularly preferred propellant is a blend of n-butane, isobutane and propane, especially when these components are present in the total propellant in amounts ranging from 44 to 64% for n-butane, 14 to 34% for isobutane and 11 to 32% for propane, and wherein the amounts of these three components total 100% of the propellant. An example of such an especially preferred propellant consists of n-butane, isobutane and propane in an approximate ratio of 54: 24: 22, respectively. Such a propellant is available as AP40 from Harp^{®} International.

Compositions according to the invention can be made in a conventional manner by first preparing a base composition, charging the base composition into the aerosol can, fitting a valve assembly into the mouth of the can, thereby sealing the can, and thereafter charging propellant into the can to a desired pressure, and finally fitting an actuator on or over the valve assembly.

### Other Components

A preferred additional component of compositions of the invention is a deodorant active. These are typically antimicrobial agents active against bacterial on the skin of the human body. These serve to reduce malodour and especially useful in compositions in which the amphiphilic material is not itself an antimicrobial agent.

When employed, the level of incorporation is preferably 0.01%-5%, more preferably from 0.01-2% and most preferably from 0.03%-0.5% by weight of the total composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C.

Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as Tea Tree Oil and Thyme Oil, climbazole, octapyrox, ketoconazole, zinc pyrithione and mixtures thereof.

A preferred optional component is a preservative, such as ethyl or methyl parabens or BHT (butyl hydroxy toluene), typically in an amount of from 0.01 to 0.1% by weight of the total composition.

The invention will now be described by some examples, which do not limit the extent of the invention.

### Examples

In the following examples, examples according to the invention are indicated by numbers and comparative examples are indicated by upper case letters.

The volatile silicones used in these examples were:
DC245 = cyclopentasiloxane and
DC200 (1.5 cS) = decamethyltetrasiloxane, herein referred to as DC200.

Each of these materials is available from Dow Corning.

The amphiphilic material used in these examples is designated "lipid" and was a 60:40 blend of glycerol monolaurate and isostearyl alcohol.

The ethanol used in these examples was absolute alcohol.

The hydrocarbon propellant used in these examples was a blend of n-butane, isobutane and propane, known as AP40 and available from Harp^{®} International.

The volatile silicone used in these examples was a 15: 85 blend of DC245 and DC200, unless stated otherwise.

The compositions indicated in Table 1 were prepared by methods known in the art. The base composition was prepared first, and this was then gassed with the propellant in a conventional aerosol container. The amounts indicated are percentages by weight.

The storage stability was assessed after 3 weeks at ambient temperature .

Instability manifested itself by phase separation, the lipid component(s) typically crystallising out of the liquid phase.

**Table 1**

| Example | Ethanol | Volatile silicone | Lipid | Hydrocarbon propellant | Stability |
|---|---|---|---|---|---|
| 1 | 14 | 23.5 | 12.5 | 50 | Yes |
| A | 5 | 32.5 | 12.5 | 50 | No |
| B | 2.5 | 35 | 12.5 | 50 | No |
| 2 | 20 | 10 | 20 | 50 | Yes |

As can be seen, Examples 1 and 2, having ethanol to volatile silicone ratios of 37: 63 and 67: 33, respectively, were stable, whereas Comparative Examples A and B, having ethanol to volatile silicone ratios of 13: 87 and 6.7: 93.3, respectively, were not stable.

The examples in Table 2 were prepared in the same manner as those in Table 1. Again, the amounts indicated are percentages by weight. Each of these examples was stable at both ambient temperature and at 0°C (test periods 6 weeks and 4 weeks, respectively).

**Table 2**

| Example | Ethanol | Volatile silicone | Ethanol to volatile silicone ratio | Lipid | Hydrocarbon propellant |
|---|---|---|---|---|---|
| 3 | 24 | 18.5 | 56: 44 | 7.5 | 50 |
| 4* | 20 | 22.5 | 47: 53 | 7.5 | 50 |
| 5 | 22 | 20.5 | 52: 48 | 7.5 | 50 |
| 6* | 22 | 20.5 | 52: 48 | 7.5 | 50 |
| 7 | 22.5 | 17.5 | 56: 44 | 10 | 50 |
| 8* | 22.5 | 17.5 | 56: 44 | 10 | 50 |
| 9 | 20 | 17.5 | 53: 47 | 12.5 | 50 |
| 10* | 20 | 17.5 | 53: 47 | 12.5 | 50 |
| 11 | 27.5 | 10 | 63: 27 | 12.5 | 50 |
| 12* | 27.5 | 10 | 63: 27 | 12.5 | 50 |

| | | | | | |
|---|---|---|---|---|---|
| * For these examples, the volatile silicone was 100% DC200 and not the blend of DC245 and DC200 referred to above. | | | | | |

It will be noted that these successful examples cover a wide ratio of ethanol to volatile silicone ratios, ranging from 47: 53 to 63: 27.

## Claims

1. An antiperspirant aerosol composition comprising ethanol, amphiphilic material, volatile silicone having a vapour pressure of greater than 1 Pa at 25°C and a hydrocarbon propellant, the amphiphilic material being a mixture consisting of isostearyl alcohol and glycerol monolaurate at a ratio of from 25: 75 to 45: 55 by weight, wherein:
(i) a three-component base composition consisting of the ethanol, amphiphilic material and volatile siloxane consists of 15 to 40% amphiphilic material; 10 to 50% volatile silicone and 12 to 75% ethanol; the sum of these three components being 100%; and
(ii) the ratio of ethanol to volatile silicone is 1: 4 or greater by weight; and
(iii) the propellant comprises from 35 to 95% by weight of the total composition.

2. A composition according to claim 1, wherein the ratio of ethanol to volatile silicone is 1: 3 or greater by weight.

3. A composition according to claim 2, wherein the ratio of ethanol to volatile silicone is 1: 2 or greater by weight.

4. A composition according to any of preceding claims, wherein the propellant consists solely of hydrocarbon propellant.

5. A composition according to any of preceding claims, wherein the volatile silicone comprises greater than 90% by weight of siloxanes selected from the group consisting of hexamethyldisiloxane, octamethyltrisiiloxane, decamethyltetrasiloxane, dodecamethylpentasiloxane and decamethylcyclopentasiloxane.

6. A composition according to claim 5, wherein the volatile silicone comprises greater than 90% by weight of siloxanes selected from the group consisting of hexamethyldisiloxane, octamethyltrisiiloxane, decamethyltetrasiloxane and decamethylcyclopentasiloxane.

7. A composition according to any of preceding claims, having less than 0.1% by weight of aluminium or zirconium antiperspirant salts.

8. A composition according to any of preceding claims, comprising an antimicrobial agent.

9. A cosmetic method of attaining an antiperspirant benefit comprising the topical application of a composition according to any of claims 1 to 7.

## Patentansprüche

1. Schweißhemmende Aerosolzusammensetzung, umfassend Ethanol, amphiphiles Material, flüchtiges Silikon mit einem Dampfdruck von mehr als 1 Pa bei 25°C und ein Kohlenwasserstoff-Treibmittel, wobei das amphiphile Material eine Mischung ist, die aus Isostearylalkohol und Glycerinmonolaurat in einem Gewichtsverhältnis von 25:75 bis 45:55 besteht, wobei:
(i) eine Drei-Komponenten-Basiszusammensetzung, bestehend aus dem Ethanol, dem amphiphilen Material und dem flüchtigen Siloxan, aus 15 bis 40% amphiphilem Material, 10 bis 50% flüchtigem Silikon und 12 bis 75% Ethanol besteht; wobei die Summe dieser drei Komponenten 100% beträgt; und
(ii) das Gewichtsverhältnis von Ethanol zu flüchtigem Silikon 1:4 oder größer ist; und
(iii) das Treibmittel 35 bis 95 Gew.-% der Gesamtzusammensetzung umfasst.

2. Zusammensetzung nach Anspruch 1, wobei das Gewichtsverhältnis von Ethanol zu flüchtigem Silikon 1:3 oder größer ist.

3. Zusammensetzung nach Anspruch 2, wobei das Gewichtsverhältnis von Ethanol zu flüchtigem Silikon 1:2 oder größer ist.

4. Zusammensetzung nach einem der vorhergehenden Ansprüche, wobei das Treibmittel ausschließlich aus einem Kohlenwasserstoff-Treibmittel besteht.

5. Zusammensetzung nach einem der vorhergehenden Ansprüche, wobei das flüchtige Silikon mehr als 90 Gew.-% Siloxane umfasst, die aus der Gruppe ausgewählt sind, bestehend aus Hexamethyldisiloxan, Octamethyltrisiloxan, Decamethyltetrasiloxan, Dodecamethylpentasiloxan und Decamethylcyclopentasiloxan.

6. Zusammensetzung nach Anspruch 5, wobei das flüchtige Silikon mehr als 90 Gew.-% Siloxane umfasst, die aus der Gruppe ausgewählt sind, die aus Hexamethyldisiloxan, Octamethyltrisiloxan, Decamethyltetrasiloxan und Decamethylcyclopentasiloxan besteht.

7. Zusammensetzung nach einem der vorhergehenden Ansprüche, die weniger als 0,1 Gew.-% schweißhemmende Aluminium- oder Zirkonium-Salze enthält.

8. Zusammensetzung nach einem der vorhergehenden Ansprüche, die ein antimikrobielles Mittel umfasst.

9. Kosmetisches Verfahren zur Erzielung einer schweißhemmenden Wirkung, umfassend die topische Anwendung einer Zusammensetzung nach einem der Ansprüche 1 bis 7.

## Revendications

1. Composition de type aérosol antitranspirante comprenant de l'éthanol, une substance amphiphile, un silicone volatil ayant une pression de vapeur supérieure à 1 Pa à 25°C et un propulseur hydrocarboné, la substance amphiphile étant un mélange consistant en alcool isostéarylique et monolaurate de glycérol dans un rapport de 25:75 à 45:55 en poids, dans laquelle:
(i) une composition de base à trois composants consistant en l'éthanol, la substance amphiphile et le siloxane volatil consiste en 15 à 40 % de substance amphiphile; 10 à 50 % de silicone volatil et 12 à 75 % d'éthanol; la somme de ces trois composants étant 100 %; et
(ii) le rapport de l'éthanol au silicone volatil est 1:4 ou plus en poids; et
(iii) le propulseur représente 35 à 95 % en poids de la composition totale.

2. Composition selon la revendication 1, dans laquelle le rapport de l'éthanol au silicone volatil est 1:3 ou plus en poids.

3. Composition selon la revendication 2, dans laquelle le rapport de l'éthanol au silicone volatil est 1:2 ou plus en poids.

4. Composition selon l'une quelconque des revendications précédentes, dans laquelle le propulseur consiste seulement en propulseur hydrocarboné.

5. Composition selon l'une quelconque des revendications précédentes, dans laquelle le silicone volatil comprend plus de 90 % en poids de siloxanes choisis dans le groupe consistant en l'hexaméthyl-disiloxane, l'octaméthyltrisiiloxane, le décaméthyltétrasiloxane, le dodécaméthylpentasiloxane et le décaméthylcyclopentasiloxane.

6. Composition selon la revendication 5, dans laquelle le silicone volatil comprend plus de 90 % en poids de siloxanes choisis dans le groupe consistant en l'hexaméthyldisiloxane, l'octaméthyltrisiiloxane, le décaméthyltétrasiloxane et le décaméthylcyclopentasiloxane.

7. Composition selon l'une quelconque des revendications précédentes, ayant moins de 0,1 % en poids de sels antitranspirants d'aluminium ou de zirconium.

8. Composition selon l'une quelconque des revendications précédentes, comprenant un agent antimicrobien.

9. Procédé cosmétique d'obtention d'un bienfait antitranspirant comprenant l'application topique d'une composition selon l'une quelconque des revendications 1 à 7.
